Europäisches Patentamt

European Patent Office

Office européen des brevets

(11) Veröffentlichungsnummer: **0 533 039 B1**

## EUROPÄISCHE PATENTSCHRIFT

(45) Veröffentlichungstag der Patentschrift: **30.08.95**

(51) Int. Cl.⁶: **A23K 1/16**, C12P 13/08, C12P 13/04

(21) Anmeldenummer: **92115390.4**

(22) Anmeldetag: **09.09.92**

(54) **Tierfuttermittelsupplement auf Fermentationsbrühe-Aminosäurebasis, Verfahren zu dessen Herstellung und dessen Verwendung.**

(30) Priorität: **17.09.91 DE 4130868**

(43) Veröffentlichungstag der Anmeldung:
**24.03.93 Patentblatt 93/12**

(45) Bekanntmachung des Hinweises auf die
Patenterteilung:
**30.08.95 Patentblatt 95/35**

(84) Benannte Vertragsstaaten:
**AT BE CH DE DK ES FR GB GR IT LI LU NL PT SE**

(56) Entgegenhaltungen:
EP-A- 0 122 163
DD-A- 139 205
DE-A- 2 357 119
FR-A- 2 556 230
FR-A- 2 640 640

DATABASE WPIL Week 8642, Derwent Publications Ltd., London, GB; AN 86-275569

(73) Patentinhaber: **Degussa Aktiengesellschaft**
**Weissfrauenstrasse 9**
**D-60311 Frankfurt (DE)**

(72) Erfinder: **Binder, Wolfram, Dr.**
**Lerchenweg 2**
**W-6458 Rodenbach (DE)**
Erfinder: **Friedrich, Heinz, Dr.**
**Grünaustrasse 4**
**W-6450 Hanau 9 (DE)**
Erfinder: **Lotter, Hermann, Dr.**
**Beethovenstrasse 1**
**W-6452 Hainburg (DE)**
Erfinder: **Tanner, Herbert, Dr.**
**Wildaustrasse 20**
**W-6450 Hanau 9 (DE)**
Erfinder: **Holldorff, Henning, Dr.**
**Hadrianstrasse 9a**
**W-6472 Altenstadt (DE)**
Erfinder: **Leuchtenberger, Wolfgang, Dr.**
**Gronauer Strasse 10**
**W-4800 Bielefeld (DE)**

CHEMICAL ABSTRACTS, vol. 115, no. 17, 28. Oktober 1991, Columbus, Ohio, US; abstract no. 181567b, KAIHO, YASUNORI 'Isolation of tryptophan from fermentation broth by decanting centrifuges' Seite 767 ;Spalte 2 ;

DATABASE WPIL Week 8135, Derwent Publications Ltd., London, GB; AN 81-63134D

CHEMIE. INGENIEUR. TECHNIK Bd. 63, Nr. 4, April 1991, WEINHEIM DE Seiten 390 - 391 DETLEF SCH[RTGES ET AL. 'Aufarbeitung einer Aminos ure-L sung - Untersuchungen zum Einsatz keramischer Membranen zur Abtrennung der Biomasse'

**Beschreibung**

Die Erfindung betrifft ein neues Tierfuttermittelsupplement mit hohem Gehalt mindestens einer Aminosäure, ein Verfahren zu dessen Herstellung durch Fermentation und dessen Verwendung.

Tierfuttermittel werden mit einzelnen Aminosäuren entsprechend dem Bedarf der Tiere supplementiert. Supplementiert werden neben dem chemisch hergestellten Methionin insbesondere die fermentativ hergestellten Aminosäuren Lysin, Threonin und Tryptophan. So ist z. B. bei Schweinen das für die optimale Futterverwertung = minimaler Gülleanfall ermittelte Verhältnis dieser drei Aminosäuren Lysin : Threonin : Tryptophan = 100 % : 65 % : 18 %, wenn man Lysin = 100 % ansetzt (siehe T. C. Wang, M. Fuller, British Journal of Nutrition, 62,77-89 (1989).

Zur Supplementierung von Tierfuttermitteln z. B. mit L-Lysin wird bisher weit überwiegend das L-Lysin-Monohydrochlorid mit einem L-Lysingehalt von 80 % eingesetzt. Da das L-Lysin durch Fermentation hergestellt wird, muß es zur Herstellung des Monohydrochlorids zunächst einmal von allen übrigen Bestandteilen der rohen Fermentationsbrühe in aufwendigen Verfahrensschritten abgetrennt, dann in das Monohydrochlorid umgewandelt und letzteres zur Kristallisation gebracht werden.

Es hat daher in der Vergangenheit nicht an Versuchen gefehlt, die aufwendige Herstellung von Futter-Aminosäuren, insbesondere von reinem L-Lysin-Monohydrochlorid zu vermeiden und die rohe Fermentationsbrühe kostengünstiger in ein festes Tierfuttermittel zu überführen. Alle diese Versuche haben aber zu keinem wirtschaftlich tragbaren Ergebnis geführt. Die einfache Entwässerung der rohen Fermentationsbrühe führte zu einem stark hygroskopischen und klebrigen Konzentrat, das in dieser Form nicht als Tierfuttermittel eingesetzt werden konnte. Um zu einem rieselfähigen und lagerungsstabilen Produkt zu gelangen, mußte eine große Menge an den verschiedenartigsten Zuschlagstoffen in das Konzentrat eingemischt werden. Dadurch wurde aber der in vielen Fällen ohnehin schon relativ geringe Gehalt der Aminosäure noch weiter vermindert.

Aus der EP-B 122 163 ist schließlich ein Verfahren bekannt, nach dem bei Einhaltung ganz spezieller Fermentationsbedingungen eine rohe Fermentationsbrühe erhalten werden kann, die sich zu einem festen und stabilen Produkt eintrocknen läßt. Dieses Produkt wird wieder durch Entwässerung der gesamten Fermentationsbrühe erhalten, sein Gehalt an L-Lysin betragt jedoch nur 35 bis 48 Gewichtsprozent und liegt demnach deutlich niedriger als der des L-Lysin-Monohydrochlorids. Die im Produkt verbleibende Biomasse wirkt hierbei wie ein Zuschlagstoff zur Verbesserung der Rieselfähigkeit.

Schließlich kennt man im Stand der Technik auch Maßnahmen zur Abtrennung von Biomasse beispielsweise in klassischen industriellen Prozessen der Aminosäure-Aufarbeitung (FR-A-2 556 230, Chem. Ing. Tech. 63 (1991), Nr. 4, S. 390 - 391).

Aufgabe der Erfindung ist ein Tierfuttermittelsupplement, das mindestens eine Aminosäure in hoher Konzentration bei wenig störenden Nebenprodukten enthält. Das Supplement soll kostengünstig in der Herstellung und als Endprodukt nicht übermäßig hygroskopisch oder klebrig sein. Außerdem soll der Gehalt der Aminosäure mit einfachen Mitteln standardisierbar sein. Aufgabe ist außerdem ein Verfahren zur Herstellung des Tierfuttermittelsupplements und dessen Verwendung.

Hinsichtlich des Tierfuttermittelsupplements wird die Aufgabe gelöst mit einem Produkt gemäß Anspruch 1.

Ein erfindungsgemäßes Aminosäuretierfuttermittelsupplement enthält neben der fermentativ erhaltenen Aminosäure(n) noch den überwiegenden Teil der Inhaltsstoffe der Fermentationsbrühe, weist folgende Zusammensetzung der Trockenmasse auf:

| | |
|---|---|
| freie Aminosäure(n) | 40 bis 90 Gewichtsprozent |
| Proteine | maximal 10 Gewichtsprozent |
| Carbonsäuren mit weniger als 8 C-Atomen | maximal 8 Gewichtsprozent |
| Gesamt-Zucker | maximal 10 Gewichtsprozent |
| Fette und Öle | maximal 5 Gewichtsprozent |
| Mineralstoffe | 3 bis 30 Gewichtsprozent |

und kann direkt durch Sprühtrocknung aus der Fermentationsbrühe gewonnen werden.

Dieses Produkt enthält insbesondere noch den überwiegenden Teil der Inhaltsstoffe der Fermentationsbrühe, gegebenenfalls ausgenommen zumindest eines Teils der Biomasse bzw. allgemein der mechanisch abtrennbaren Produkte.

Üblicherweise enthält das Produkt einen Restwassergehalt von mind 0.5 Gew.%, um ein Verklumpen zu vermeiden, sollte der Wassergehalt jedoch 5 Gew.-% nicht überschreiten.

Der Proteingehalt wird ermittelt aus dem Gesamtstickstoffgehalt minus anorganischem Stickstoffgehalt minus Stickstoffgehalt der freien Aminosäuren und Multiplikation des erhaltenen Wertes mit 6,25 ((Ges. N % - anorg. N % - AS N %) $\cdot$ 6,25). Der Gesamtstickstoffgehalt wird bestimmt durch Aufschluß nach Kjeldahl (Standardverfahren), der anorganische Stickstoffgehalt (Ammonium-Gehalt) z.B. wird bestimmt colorimetrisch, titrimetrisch oder potentiometrisch, der Stickstoffgehalt der freien Aminosäure wird durch die quantitative Bestimmung der freien Aminosäuren (Aminosäureanalysator (ASA)) mit anschließender Berechnung des N-Gehalts ermittelt. Als Mineralstoffe wird die Gesamtheit aller anorganischer Kationen und Anionen bezeichnet.

Basische Aminosäuren liegen als Salze vor und haben deshalb einen höheren minimalen Gehalt an Mineralstoffen (i. d. R. mind. 10 %) und einen niedrigeren maximalen Gehalt an freier Aminosäure (i. d. R. max. 72 %, insbesondere max. 67 %). Der maximale Wert wird hier durch das Anion (z.B. $SO_4^{2-}$ ; $Cl^-$) bestimmt, als Regel kann der obere Wert bei basischen Aminosäuren zu 90 % des theoretischen Gehalts der freien Aminosäurebase im Salz gesetzt werden (Beispiel: $(Lys)_2 \cdot H_2SO_4$ enthält 75 Gew. % Lys, ein entsprechendes erfindungsgemäßes Produkt enthält max 75 0,9 = 67,5 Gew. % Lys. ). Der Gehalt wird zudem noch von den verwendeten Mikroorganismen und den eingesetzten Fermentationmedien bestimmt.

Hohe Aminosäuregehalte bis max. 90 % i. d. T sind beispielsweise bei der Herstellung von Threonin mit Escherichia coli BKIIM B-3996 (FR-A 26 40 640), hier insbesondere wegen der relativ einfachen Medien, erreichbar.

Bei der Herstellung von Lysin bzw. Tryptophan mit Corynebacterium glutamicum, bzw. Escherichia coli K12-Abkömmlingen liegt der Aminosäuregehalt i. d. R. etwas niedriger, insbesondere bei Tryptophan sind fermentativ derzeit maximale Gehalte von 70 Gew.% typisch.

Auf Fermentationsbruhe-Aminosäurebasis heißt, daß zumindest ein Teil (vorzugsweise der überwiegende Teil) des Supplements die Trockenmasse einer Fermentationsbrühe ist, ausgenommen der durch mechanische Trenntechniken entfernbaren Stoffe, die gemäß der Ansprüche entfernt sein können.

Mechanisch entfernbare Stoffe sind insbesondere die Biomasse und Proteine. Mechanische Trenntechniken sind insbesondere Filtration (z. B. Ultra- und Mikrofiltration), Separation (z. B. Zentrifugation und Dekantieren), wobei im wesentlichen nur ungelöste und/oder höhermolekulare Stoffe abgetrennt werden.

Zur Erfindung gehören also solche Tierfuttermittelsupplemente, die neben einer oder mehrer Aminosäuren noch den überwiegenden Teil der Inhaltsstoffe der zu den Aminosäure(n) gehörigen Fermentationsbrühe, ausgenommen zumindest eines Teils der Biomasse, enthalten; sowie solche Tierfuttermittelsupplemente, die obiger Zusammensetzung genügen, wobei hier die Biomasse vollständig, teilweise oder gar nicht enthalten sein kann. Insbesondere wenn zumindest ein Teil der Biomasse noch im Supplement enthalten ist, dann sollten die Mikroorganismen vorteilhaft natürliche Mikroorganismen sein, d. h. solche, die keiner Genmanipulation unterzogen wurden bzw. nicht unter das deutsche Gentechnikgesetz fallen.

Die erfindungsgemäßen Supplemente lassen sich direkt, z. B. durch (Sprüh)trocknung einer Fermentationsbrühe gewinnen, gegebenenfalls unter vorheriger Abtrennung zumindest eines Teils der Biomasse. Überraschend und von besonderem Vorteil ist dabei, daß die Supplemente mit einem gegenüber bisher bekannten Produkten (aus Fermentationsbrühen) niedrigeren Gehalt an Protein auskommen und trotzdem zu einem handhabbarem Pulver oder Granulat verarbeitet (getrocknet) werden können.

Bei sehr sauberen (an organischen Substanzen rückstandsarmen) Fermentationen kann die Brühe sogar ohne die Biomasse und im wesentlichen ohne Hilfsstoffe wie zusätzliche (mineralische) Trägerstoffe zu einem handhabbaren Granulat getrocknet werden. Ebenso können Fermentationsbrühen, die von vornherein so geführt werden, daß nur wenig Biomasse in ihnen enthalten ist, direkt (im wesentlichen ohne Hilfsstoffe wie zusätzliche (mineralische) Trägerstoffe) zum Supplement getrocknet werden. Beide Wege führen zu den erfindungsgemäßen proteinreduzierten bzw. proteinarmen Supplementen. Bekannte Produkte enthalten immer große Mengen an Biomasse und oft noch erhebliche Mengen an weiteren Trägerstoffen.

Der Proteingehalt des Supplements ist sehr wichtig, vorteilhaft soll er 10 Gew.-% nicht überschreiten, vorzugsweise liegt er unter 10 Gew.-%, insbesondere bei max. 7 Gew.-%. Ganz bevorzugt sind Proteingehalte unter 5 Gew.-%. Aus prozeßökonomischen Gründen liegt der Proteingehalt meist bei mindestens 0,5 %, in der Regel bei 1 Gew.-% und darüber. Der niedrige Proteingehalt ist insofern bedeutend, da mit dem Supplement der Stickstoff bzw. Proteingehalt des Mischfutters möglichst wenig erhöht werden soll. Schon eine Reduzierung des N-Gehalts um 0,05 % ist bedeutend. Mit der Proteinerhöhung im Futter steigt der Bedarf an Supplement sowie der N-Gehalt in der Gülle und sinkt der prozentuale Anteil der Supplementaminosäure(n) im Produkt, wodurch u.a. höhere Frachtkosten bezogen auf den Supplementgehalt verursacht werden.

Insbesondere die Gülle kann zu gravierenden Umweltproblemen führen. Ein hoher Proteingehalt muß außerdem bei der Proteinanalyse berücksichtigt werden. Es gilt daher möglichst wenig unnötiges und von der Zusammensetzung her unbekanntes Protein ins Mischfutter einzubringen.

Üblicherweise enthält die Fermentationsbrühe über 10 - 20 Gew.-% Biomasse i. d. T. Wenn der Proteingehalt im Tierfuttersupplement zu hoch wird, wird der Anteil der Fermentationsbiomasse im Supplement reduziert, vorzugsweise i. d. T. auf max. 10 Gew.% begrenzt. Häufig liegt der Anteil der Fermentationsbiomasse bei maximal 5 Gew.-%. Bevorzugt sind auch biomassefreie Supplementzusammensetzungen, das heißt solche mit höchstens 0,1 Gew.-% Biomasse.

Vorzugsweise liegt im Supplement überwiegend nur eine Aminosäure vor.

Ein solches Supplement ist dann universell entsprechend seinem Aminosäure-Gehalt zu jedem Futter oder zu Prämixen zudosierbar. Bei definierten Futtermischungen kann es aber auch zweckmäßig sein, daß mehrere Aminosäuren in dem Supplement in einem bestimmten Verhältnis zueinander vorliegen, so daß mit nur einem Supplement die gewünschte Aminosäure-Supplementierung erreicht wird. Das Verhältnis dieser Aminosäuren kann beispielsweise durch Mischen mehrerer Aminosäure-Fermentationsbrühen oder Supplemente (mit aller oder reduzierter oder ohne Biomasse), sowie auch durch Zudosieren vorteilhaft geringer Mengen reiner Aminosäure (z. B. Trp und/oder Thr zu z. B. einem Lysin-Supplement) erhalten werden.

In der vorliegenden Erfindung sind geeignete Aminosäuren insbesondere Lysin, Threonin, Tryptophan sowie Leucin, Isoleucin, Valin, Prolin und Alanin.

Neben einer einzelnen Aminosäure oder mehreren gezielt enthaltenen bzw. definierten Aminosäuren im Supplement sollten möglichst wenig weitere undefinierte oder nicht zur Supplementierung geeignete Aminosäuren im Supplement vorhanden sein.

Die Supplementaminosäuren sollten mindestens 95 Gew.-% der Gesamtmenge der freien Aminosäuren im Supplement ausmachen, vorzugsweise liegt der Gehalt bei mindestens 97 Gew.-%. Aus prozessökonomischen Gründen ist die Untergrenze der anderen, bei der Supplementierung nicht zu berücksichtigenden Aminosäuren bzw. nicht deklarierten (undefinierten) $\alpha$-Aminosäuren meist bei 0,5 Gew.-%, üblicherweise liegt deren Gehalt bei mindestens 1 Gew.-%. Diese anderen Aminosäuren haben ähnliche Nachteile wie der Peptidgehalt, sie sind aber bei einer Gesamtaminosäurenanalyse leichter bestimmbar.

Die Summe der anderen $\alpha$-Aminosäuren und Proteine sollte vorzugsweise nicht über 11 Gew.-% liegen, üblich sind unter 11 Gew.-% liegende Werte, insbesondere ein Gehalt von 8 Gew.-% und darunter. Die Untergrenze liegt hier bei üblicherweise 2 Gew.-%.

Die angegebenen Werte für Proteine und Aminosäuren bzw. deren Summe sind so gering, daß diese Anteile bei der Protein/Aminosäure-Analyse des zu supplementierenden Futters nicht berücksichtigt werden müssen, höhere Werte können unter Umständen die mit der Supplementierung gewünschte optimale Aminosäure-Zusammensetzung beeinträchtigen.

Der Gesamtzuckergehalt beinhaltet die von den Mikroorganismen verwertbaren und nicht verwertbaren Zucker, vorteilhaft liegt er bei maximal 5 Gew.-%, besonders günstig sind Werte bei 2,5 Gew.-% und darunter, die besten Gehalte liegen bei maximal 1,5 Gew.-%. Je höher der Zuckergehalt ist, desto hykoskopischer kann das Produkt sein und zum Verkleben neigen. Außerdem können bei höheren Zuckergehalten in der Eindampfung und Trocknung Maillard-Produkte entstehen, die die Ausbeute und Produkteigenschaften mindern. Der Zuckergehalt kann dadurch gesenkt werden, daß man zumindest gegen Ende der Fermentation die Zuckerzufuhr begrenzt.

Der Anteil an Fetten und Ölen wird vorteilhaft üblicherweise auf 3 Gew.-% begrenzt, wie bei den Proteinen sollte auch hier der Anteil nicht so hoch sein, um bei der Supplementierung des Mischfutters unberücksichtigt bleiben zu können.

Für die Supplementierung sind besonders Supplemente geeignet, die einen Gehalt der gewünschten freien Aminosäure(n) von mindestens 49,5 Gew.-% aufweisen, vorzugsweise liegt der Wert bei 50 % oder darüber. Die obere Grenze liegt vorzugsweise aus prozessökonomischen Gründen bei basischen Aminosäuren bei maximal 60 Gew.-%, Werte von maximal 57 Gew.-% haben sich als besonders geeignet ausgestellt; bei neutralen Aminosäuren sind diese Werte 85 Gew.-% bzw. 80 Gew.-%.

Die Supplemente können wie durch (Sprüh)trocknung der Fermentationsbrühe (ggf. mit reduzierter Biomasse) erhalten, in Mischungen solcher Supplemente (auch der Brühen) oder mit weiteren Zusätzen versehen als Prämixe wie Spurenelement-/Vitamin-Prämixe oder Mineralstoff-Prämixe eingesetzt werden.

Solche Supplemente sind gegenüber der Reinisolation sowie gegenüber bisherigen Produkten aus Fermentationsbrühen preiswerter bei hohem Gehalt, sind leicht standardisierbar und enthalten wenig Nebenprodukte bei geringen Entstehungskosten.

Die beschriebene Supplementzusammensetzung ist insbesondere für die Lysin-Supplementierung geeignet, da Lysin fermentativ leicht zugänglich ist und eine der wichtigsten Supplement-Aminosäuren ist.

Das erfindungsgemäße Tierfuttermittel ist meist ein hellbeiges bis braunbeiges Pulver mit einer Schüttdichte zwischen 0,4 und 0,7 kg/l. Im Fall von L-Lysin liegt dieses in salzartiger Bindung vor, zum Beispiel als Sulfat oder Carbonat. Es ist völlig stabil und nach einer Lagerung über 12 Monate bei 25 °C ist keine Abnahme des Gehaltes an L-Lysin feststellbar. Sein Hygroskopizitätsverhalten ist gut.

Es zeigt eine gute Rieselfähigkeit, neigt nicht zur Verklumpung und läßt sich leicht homogen in Vollfuttermittel oder Vormischungen einmischen. Es ist auch als Bestandteil solcher Mischungen von ausgezeichneter Stabilität.

Als Quelle für L-Lysin hat es, bezogen auf gleichen Gehalt an L-Lysin, mindestens die gleiche Wirksamkeit wie das üblicherweise verwendete reine L-Lysin-Monohydrochlorid.

Ein weiterer Gegenstand der Erfindung sind Verfahren zur Herstellung dieser Tierfuttermittelsupplemente. Die Verfahren sind dadurch gekennzeichnet, daß man einen mindestens eine Aminosäure produzierenden Mikroorganismus in einem Fermentationsmedium kultiviert, das mindestens eine Kohlenstoffquelle, mindestens eine Stickstoffquelle, Mineralsalze und Spurenstoffe enthält, wobei man die Fermentation so führt, daß an ihrem Ende eine rohe Fermentationsbrühe mit einem Gehalt an verwertbarem Zucker von max. 4 g/l und insbesondere mit einem Feststoffgehalt von 7,5 bis 26 Gewichtsprozent, einem Aminosäuregehalt von 1-20 Gewichtsprozent, vorteilhaft von 4 bis 10,5 Gewichtsprozent und einem Zuckergehalt von maximal 2,6 Gewichtsprozent erhalten wird. Alternativ kann dabei die Fermentation so kultiviert (gegebenenfalls unter Verwendung eines geeigneten Mikroorganismus) werden, daß der Gehalt an Biomasse so limitiert ist, daß die Trockenmasse der Fermentationsbrühe max. 10 Gew.-% an Protein und insbesondere 40 - 90 Gew.-% an Aminosäure(n), max. 8 Gew.-% an Carbonsäuren mit weniger als 8 C-Atomen, max. 10 Gew.-% an Gesamtzucker, max. 5 Gew.% an Fette und Öle und max. 5 - 30 Gew.-% an Mineralstoffe enthält, oder es wird vorzugsweise nach Ende der Fermentation zumindest teilweise die Biomasse insbesondere auf max. 10 Gew.-% i. d. T. reduziert, bzw. die die Biomasse bildenden Mikroorganismen und ggf. andere Stoffe werden durch mechanische Trenntechniken und unter Belassen der überwiegenden Anteile der restlichen Komponenten der Fermentationsbrühe vorzugsweise nach Ende der Fermentation entfernt. Diese Verfahren können miteinander kombiniert werden. Vorteilhaft wird die verbleibende Brühe eintrocknet. Das Eintrocknen erfolgt vorteilhafterweise durch Eindicken der verbliebenen Brühe auf einen Feststoffgehalt von 40 bis 60 Gew.-% und Trocknen dieser viskosen Brühe, z.B. durch Sprühtrocknung. Wenn keine Biomasse abgetrennt werden soll, ist die Fermentation so zu führen, daß möglichst wenig Biomasse produziert wird, wobei die zugegebenen Nährstoffe am Ende möglichst weitgehend verbraucht sein sollen. Eine solche Fermentation ist z.B. in der DE-A 41 30 867, Beispiel 3, beschrieben.

Vorteilhafterweise wird die Fermentation so geführt, daß über mindestens 30 %, vorzugsweise über 70 % der Fermentationsdauer die Konzentration an verwertbarem Zucker in der Fermentationsbrühe höchstens 0,3 Gew.-% beträgt.

Gegen Ende der Fermentation wird der Gehalt an verwertbarem Zucker auf maximal 1 g/l eingestellt, das heißt es wird kein Zucker zugegeben und die Fermentation erst bei Erreichen oder Unterschreiten dieses Wertes abgebrochen. Normalerweise wird der Fermentationsrückstand nach Entfernen zumindest eines Teils der Biomasse auf einen Wassergehalt von maximal 5 Gew.-% getrocknet.

Vorteilhaft kann die Biomasse vollständig entfernt werden, das erhaltene Endprodukt hat dann einen besonders hohen Gehalt an Lysinbase bzw. anderer Aminosäuren. Außerdem ist das Produkt, das durch vollständige Entfernung der Biomasse erhalten wird, besonders geeignet zur Standardisierung eines Endproduktes auf einen konstanten Aminosäuregehalt. Dies ist insbesondere dann notwendig, wenn bei einzelnen Fermentationschargen unterschiedliche Aminosäuregehalte erreicht werden.

Als Aminosäure produzierende Mikroorganismen werden für Lysin bevorzugt geeignete Mutanten der Species Corynebacterium oder Brevibacterium verwendet, z. B. der bei der Deutschen Sammlung für Mikroorganismen unter DSM 5715 hinterlegte und frei erhältliche Stamm. Als Kohlenstoffquelle werden vorzugsweise Stärkehydrolysate (Glucose) oder Saccharose eingesetzt. Ein geringer Anteil kann auch aus Zuckerrüben- oder Zuckerrohrmelasse stammen. Dieser Anteil sollte 5 Gew.-% der Gesamtkohlenstoffquelle nicht überschreiten (≙ 10 Gew.-% Melasse in der Gesamtkohlenstoffquelle).

Für Threonin und Tryptophan werden bevorzugt geeignete Mutanten der Species Escherichia coli z.B. für Threonin der Stamm BKIIM B-3996 (FR-A 2 640 640) eingesetzt.

Als Stickstoffquelle dienen neben Ammoniak oder Ammoniumsulfat bevorzugt Hydrolysate von Protein enthaltenden Stoffen, wie Maiskleber, Sojamehl oder der Biomasse aus einem vorhergehenden Ansatz. Andere geeignete Stickstoffquellen sind beispielsweise Maisquellwasser oder Fischpepton.

Die Fermentationstemperatur liegt zweckmäßigerweise zwischen 30 und 40°C, der pH-Wert des Fermentationsmediums zwischen 6,0 und 8,0. Die Dauer der Fermentation beträgt im allgemeinen maximal 100 Stunden.

Nach dem Ende der Fermentation können die Mikroorganismen thermisch oder auch durch andere Verfahren, z. B. durch Zusatz einer Mineralsäure, z. B. Schwefelsäure, abgetötet werden. Gegebenenfalls ein Teil der Biomasse wird anschließend nach bekannten Verfahren, wie Separieren, Dekantieren, einer Kombination von Separieren und Dekantieren, Ultrafiltration oder Mikrofiltration, abgetrennt. Die von der Biomasse zumindest teilweise befreite Brühe wird dann durch bekannte Verfahren, z. B. in einem Dünnschicht- oder Fallfilmverdampfer zu einem Vorkonzentrat mit einem Feststoffgehalt von 40 bis 60 Gewichtsprozent eingedickt. Zum endgültigen Eintrocknen des Vorkonzentrates kann beispielsweise ein Sprühtrockner, ein Spin-flash-Trockner oder ein Wirbelschichttrockner dienen.

Die abgetrennte Biomasse kann auf einfache Weise mittels Schwefelsäure, Salzsäure oder geeigneten Enzymen hydrolysiert werden. Das so erhaltene Hydrolysat kann mit Vorteil bei einer nachfolgenden Fermentation dem Fermentationsmedium als Stickstoffquelle zugesetzt werden. Durch die Recyclierung der Biomasse werden Rohstoffe eingespart.

Sofern eine Standardisierung des erfindungsgemäßen Tierfuttermittels bezüglich des Gehaltes der Aminosäure, insbesondere L-Lysin erwünscht ist, kann dies beispielsweise durch entsprechende Wahl der Menge an verbleibender Biomasse und/oder geeignetes Abmischen der Vorkonzentrate aus verschiedenen Fermentationsbrühen erfolgen. Diese Vorkonzentrate sind besonders geeignet, wenn sie keine Biomasse mehr enthalten, da diese Produkte homogen sind.

Entsprechend können auch biomassefreie oder -reduzierte Brühen mit Originalbrühen zur Standardisierung gemischt werden. Eine andere Möglichkeit ist der Zusatz geringer Mengen an futtermittelrechtlich unbedenklichen Zuschlagstoffen, beispielsweise Weizenkleie oder Maisspindelmehl. Auch eine Kombination der einzelnen Schritte untereinander ist möglich.

Die nachfolgenden Beispiele sollen die Erfindung näher erläutern:

Beispiel 1:

In einem Fermentationsbehälter mit Rührwerk und Belüftungssystem wurden 150 kg einer sterilen Lösung folgender Zusammensetzung vorgelegt:

```
Wasser                               130 l
Glucose                             12,4 kg
Maiskleberhydrolysat, schwefelsauer  9,0 kg
Ammoniumsulfat                       1,5 kg
Mineralsalze          ⎤
                      ⎬  0,4 kg
Spurenstoffe          ⎦
mit einer Ammoniaklösung auf einen pH-Wert von 7,5
eingestellt.
```

Dieser Lösung wurden bei 33 bis 35°C 12 l einer im gleichen Fermentationsmedium, aber in einem separaten Fermentationsbehälter angezogenen Impfkultur eines Corynebacteriums zugesetzt.

Innerhalb von 40 Stunden wurden 77 l einer sterilen Lösung zudosiert, die vor der Neutralisation auf pH 7,5 folgende Zusammensetzung hatte:

```
Wasser                               38 l
Glucose                             40,0    kg
Maiskleberhydrolysat, schwefelsauer  8,3    kg
Ammoniumsulfat                       0,9    kg
Entschäumer (Nalco®)                 0,08 kg
Mineralsalze          ⎤
                      ⎬  0,2    kg
Spurenstoffe.         ⎦
```

Während der gesamten Fermentationsperiode wurde der pH-Wert mittels Ammoniaklösung zwischen 7,0 und 7,5 gehalten. Die Rührerdrehzahl wurde auf 600 Upm und die Belüftungsrate auf 0,5 bis 0,7 vvm eingestellt.

Am Ende der Fermentationsperiode wurden 275 kg einer rohen Fermentationsbrühe mit einem Feststoffgehalt von 34,1 kg, einem Gehalt an L-Lysinbase von 15,5 kg und einem Gehalt an Zucker von 0,71 kg erhalten. Die Mikroorganismen wurden thermisch abgetötet und mit einer Kombination von Separator und Dekanter abgetrennt.

Die von der Biomasse befreite Brühe wurde in einem Fallfilmverdampfer unter vermindertem Druck auf einen Feststoffgehalt von etwa 52 Gewichtsprozent eingedickt.

Anschließend wurde dieses Vorkonzentrat in einem Sprühtrockner zu einem hellbraun-beigem Pulver mit einem Schüttgewicht von 0,51 kg/l und mit folgender Zusammensetzung entwässert:

| | |
|---|---|
| L-Lysinbase | 52,1 Gewichtsprozent |
| andere α-Aminosäuren | 2,5 Gewichtsprozent |
| Proteine | 8,8 Gewichtsprozent |
| Carbonsäuren mit weniger als 8 C-Atomen | 6,8 Gewichtsprozent |
| Zucker | 2,4 Gewichtsprozent |
| Fette und Öle | 2,5 Gewichtsprozent |
| Mineralstoffe | 21,1 Gewichtsprozent |
| Wasser | 1,6 Gewichtsprozent. |

Beispiel 2:

Die Fermentation wurde durchgeführt wie im Beispiel 1 mit dem Unterschied, daß anstelle des reinen schwefelsauren Maiskleberhydrolysats ein Gemisch aus 3,5 kg schwefelsaurem Maiskleberhydrolysat und 13,8 kg schwefelsaurem Hydrolysat der im Beispiel 1 abgetrennten Biomasse eingesetzt wurde.

Am Ende der Fermentationsperiode wurden 272 kg einer rohen Fermentationsbrühe mit einem Feststoffgehalt von 36,2 kg, einem Gehalt an L-Lysinbase von 17,1 kg und einem Gehalt an Zucker von 0,55 kg erhalten.

Die Biomasse wurde ohne Vorbehandlung durch Ultrafiltration (≤ 300 000 Dalton) abgetrennt. Die von der Biomasse befreite Brühe wurde wie im Beispiel 1 auf einen Feststoffgehalt von etwa 53 Gewichtsprozent eingedickt.

Anschließend wurde ein Teil dieses Vorkonzentrats in einem Wirbelschichttrockner zu einem hellbraun-beigem Pulver mit einem Schüttgewicht von 0,53 kg/l und mit folgender Zusammensetzung entwässert:

| | |
|---|---|
| L-Lysinbase | 62,1 Gewichtsprozent |
| andere α-Aminosäuren | 1,2 Gewichtsprozent |
| Proteine | 2,6 Gewichtsprozent |
| Carbonsäuren mit weniger als 8 C-Atomen | 3,1 Gewichtsprozent |
| Zucker | 2,0 Gewichtsprozent |
| Fette und Öle | 1,7 Gewichtsprozent |
| Mineralstoffe | 24,4 Gewichtsprozent |
| Wasser | 1,6 Gewichtsprozent. |

Ein weiterer Teil des Vorkonzentrats wurde vor der Entwässerung im Wirbelschichttrockner mit soviel Maisspindelmehl versetzt, daß das erhaltene Festprodukt einen standardisierten Gehalt an L-Lysinbase von 60 Gewichtsprozent hatte.

Beispiel 3

In einem Fermentationsbehälter mit Rührwerk und Belüftungssystem wurden 150 kg einer sterilen Lösung folgender Zusammensetzung vorgelegt:

```
Wasser                                      133,0  kg

Melasse                                       0,77 kg

Saccharose                                    3,68 kg
```

```
Maiskleberhydrolysat, schwefelsauer  1,03 kg

Biomassehydrolysat, schwefelsauer     9,4  kg

Ammoniumsulfat                        1,3  kg

Mineralsalze                        ⎫
                                    ⎬ 0,4  kg
Spurenstoffe                        ⎭

mit einer Ammoniaklösung auf einen pH-Wert von 7,5

eingestellt.
```

Dieser Lösung wurden bei 33 bis 35°C 17,6 l einer im gleichen Fermentationsmedium, aber in einem separaten Fermentationsbehälter angezogenen Impfkultur eines Corynebacteriums mit der Stamm-Nummer DSM 5715 zugesetzt. Der Stamm ist bei der Deutschen Sammlung für Mikroorganismen frei erhältlich.

Innerhalb von 39 Stunden wurden 78 l einer sterilen Lösung zudosiert, die vor der Neutralisation auf pH 7,5 folgende Zusammensetzung hatte:

```
Wasser                                      36   kg

Melasse                                      2,7  kg

Saccharose                                  43,1  kg

Maiskleberhydrolysat, schwefelsauer 1,1  kg

Biomasse hydrolysat, schwefelsauer 10,7  kg

Ammoniumsulfat                               0,9  kg

Mineralsalze                        ⎫
                                    ⎬ 0,2 kg
Spurenstoffe.                       ⎭
```

Während der gesamten Fermentationsperiode wurde der pH-Wert mittels Ammoniaklösung zwischen 7,0 und 7,5 gehalten. Die Rührerdrehzahl wurde auf 600 Upm und die Belüftungsrate auf 0,5 bis 0,7 vvm eingestellt.

Am Ende der Fermentationsperiode wurden 218 kg einer rohen Fermentationsbrühe mit einem Feststoffgehalt von 29,2 kg, einem Gehalt an L-Lysinbase von 10,6 kg und einem Gehalt an Zucker von 1,4 kg erhalten. Die Mikroorganismen wurden thermisch abgetötet und mit einer Kombination von Separator und Dekanter abgetrennt.

Die von der Biomasse befreite Brühe wurde in einem Fallfilmverdampfer unter vermindertem Druck auf einen Feststoffgehalt von etwa 52 Gewichtsprozent eingedickt. Anschließend wurde dieses Vorkonzentrat in einem Sprühtrockner zu einem hellbraun-beigem Pulver mit einem Schüttgewicht von 0,50 kg/l und mit folgender Zusammensetzung entwässert:

| L-Lysinbase | 41,8 Gew.% |
|---|---|
| andere α-Aminosäuren | 4,1 Gew.% |
| Proteine | 8,5 Gew.% |
| Carbonsäuren mit weniger als 8 C-Atomen | 6,7 Gew.% |
| Zucker | 5,7 Gew.% |
| Fette und Öle | 2,8 Gew.% |
| Mineralstoffe | 25,6 Gew.% |
| Wasser | 3,0 Gew.%. |

Beispiel 4

In einem Fermentationsbehälter mit Rührwerk und Belüftungssystem wurden 6,6 kg einer sterilen Lösung folgender Zusammensetzung vorgelegt:

| Wasser | 6,05 kg |
|---|---|
| Glucose | 150 g |
| Ammoniumsulfat | 50 g |

| Mineralsalze | } 50 g |
|---|---|
| Spurenstoffe | |

mit einer Ammoniaklösung (25 %) auf einen pH-Wert von 6,5 eingestellt.

Dieser Lösung wurden bei 33° C 0,7 kg einer im gleichen Fermentationsmedium, aber in einem separaten Fermentationsbehälter angezogenen Impfkultur eines Escherichia coli K12-Abkömmlings zugesetzt.

Innerhalb von 24 Stunden wurden 1,7 kg einer sterilen Lösung folgender Zusammensetzung zudosiert:

| Wasser | 0,6 kg |
|---|---|
| Glucose | 1,1 kg |

Während der gesamten Fermentationsperiode wurde der pH-Wert mittels Ammoniaklösung bei 6,5 gehalten.

Am Ende der Fermentationsperiode wurden mehrere so erhaltene Fermentationsbrühen vereinigt und die Biomasse durch Ultrafiltration (< 300 000 Dalton) abgetrennt.

Die von der Biomasse befreite Brühe wurde in einem Lyophilisator zu einem hellbraunen Pulver folgender Zusammensetzung entwässert:

| L-Tryptophan | 46,0 Gew.-% |
|---|---|
| andere α-Aminosäuren | 4,0 Gew.-% |
| Proteine | 1,7 Gew.-% |
| Carbonsäuren mit weniger als 8 C-Atomen | 3,0 Gew.-% |
| Zucker | 1,0 Gew.-% |
| Mineralstoffe | 25,0 Gew.-% |
| Wasser | 2,3 Gew.-% |
| Nicht spezifiert | 17,0 Gew.-% |

Beispiel 5

Ein Threonintierfuttersupplement kann wie folgt erhalten werden:

Analog wie im FR-A 2 640 640 beschrieben, wird durch Kultivieren des Stammes Escherichia coli BKIIM B-3996 in einem Fermentationsmedium eine Fermentationsbrühe mit 85 g/l L-Threonin hergestellt.

Die Biomasse aus dem gentechnisch veränderten Mikroorganismus wird anschließend durch Ultrafiltration abgetrennt und die von der Biomasse befreite Brühe in einem geeigneten Trockner entwässert.

**Patentansprüche**

1.  Aminosäuretierfuttermittelsupplement auf Fermentationsbrühebasis,
    **dadurch gekennzeichnet,**
    daß das Aminosäuresupplement noch den überwiegenden Teil der Inhaltsstoffe der Fermentationsbrühe enthäl die Trockenmasse folgende Zusammensetzung aufweist:

| Aminosäure(n) | 40 - 90 Gew.-% |
|---|---|
| Proteine max. | 10 Gew.-% |
| Carbonsäuren mit weniger als 8 C-Atomen max. | 8 Gew.-% |
| Gesamtzucker max. | 10 Gew.-% |
| Fette und Öle max. | 5 Gew.-% |
| Mineralstoffe | 3 - 30 Gew.-%, |

    und daß das Aminosäuresupplement direkt durch Sprühtrocknung aus der Fermentationsbrühe gewonnen werden kann.

2.  Supplement nach Anspruch 1,
    **dadurch gekennzeichnet,**
    daß ein Teil der Biomasse entfernt wurde.

3.  Supplement nach einem der vorhergehenden Ansprüche,
    **dadurch gekennzeichnet,**
    daß der Wassergehalt bei 0,5 - 5 Gew.-% liegt.

4.  Supplement nach einem der vorhergehenden Ansprüche,
    **dadurch gekennzeichnet,**
    daß es in der Trockenmasse bis zu 10 Gew.-% an Fermentationsbiomasse enthält.

5.  Supplement nach einem der vorhergehenden Ansprüche,
    **dadurch gekennzeichnet,**
    daß der Gehalt an der/n fermentativ hergestellten Aminosäure/n mindestens 49,5 Gew.-% beträgt.

6.  Supplement nach einem der vorhergehenden Ansprüche,
    **dadurch gekennzeichnet,**
    daß der Gehalt an der fermentativ hergestellten Aminosäure max. 60 Gew.-% im Falle basischer Aminosäure oder 85 Gew.-% bei neutralen Aminosäuren beträgt.

7.  Verfahren zur Herstellung eines Aminosäuretierfuttermittelsupplements auf Fermentationsbrühebasis, mit den Schritten
    - Kultivieren eines mindestens eine $\alpha$-Aminosäure produzierenden Mikroorganismus in einem Fermentationsmedium, das mindestens eine Kohlenstoffquelle, mindestens eine Stickstoffquelle, Mineralsalze und Spurenstoffe enthält,
    - Führen der Fermentation so, daß an ihrem Ende eine rohe Fermentationsbrühe mit einem Gehalt an verwertbarem Zucker von max. 4 g/l erhalten wird,
    - zumindest teilweises Reduzieren der die Biomasse bildenden Mikroorganismen insbesondere auf max. 10 Gew.-% in der Trockenmasse,
    so daß das Aminosäuretierfuttermittelsupplement direkt durch Sprühtrocknung aus der Fermentationsbrühe gewonnen werden kann.

8. Verfahren zur Herstellung eines Aminosäuretierfuttermittelsupplements auf Fermentationsbrühebasis, mit den Schritten

- Kultivieren eines mindestens eine α-Aminosäure produzierenden Mikroorganismus in einem Fermentationsmedium, das mindestens eine Kohlenstoffquelle, mindestens eine Stickstoffquelle, Mineralsalze und Spurenstoffe enthält,

- Führen der Fermentation so, daß an ihrem Ende eine rohe Fermentationsbrühe mit einem Gehalt an verwertbarem Zucker von max. 4 g/l erhalten wird,

- zumindest teilweises Reduzieren der die Biomasse bildenden Mikroorganismen insbesondere auf max. 10 Gew.-% in der Trockenmasse und gegebenenfalls anderer Stoffe durch mechanische Trenntechniken und Belassen der überwiegenden Anteile der restlichen Komponenten der Fermentationsbrühe im Supplement,

so daß das Aminosäuretierfuttermittelsupplement direkt durch Sprühtrocknung aus der Fermentationsbrühe gewonnen werden kann.

9. Verfahren zur Herstellung eines Aminosäuretierfuttermittelsupplements auf Fermentationsbrühebasis nach einem der Ansprüche 7 oder 8,
bei dem die Fermentation so geführt wird, daß an ihrem Ende eine rohe Fermentationsbrühe mit einem Gehalt an verwertbarem Zucker von max, 4 g/l und mit einem Gehalt an Biomasse erhalten wird, der so limitiert ist, daß die Trockenmasse der Fermentationsbrühe max. 10 Gew.-% an Protein und insbesondere 40 - 90 Gew.-% an Aminosäure(n), max. 8 Gew.-% an Carbonsäuren mit weniger als 8 C-Atomen, max. 10 Gew.-% an Gesamtzucker, max. 5 Gew.-% an Fette und Öle und 3 - 30 Gew.-% an Mineralstoffe enthält.

10. Verfahren nach einem der Ansprüche 7 - 9,
**dadurch gekennzeichnet,**
daß am Ende in der rohen Fermentationsbrühe ein Feststoffgehalt von 7,5 - 26 Gew.-% vorliegt.

11. Verfahren nach einem der Ansprüche 7 - 10,
**dadurch gekennzeichnet,**
daß die Fermentationsbrühe anschließend getrocknet wird auf einen Wassergehalt ≦ 5 Gew.-%.

12. Verfahren nach einem der Ansprüche 7 oder 11,
**dadurch gekennzeichnet,**
daß die Fermentation so geführt wird, daß an ihrem Ende in der rohen Fermentationsbrühe die Aminosäure(n) zu 1 - 20 Gew.-% vorliegt/en.

13. Verfahren nach einem der Ansprüche 7 bis 12,
**dadurch gekennzeichnet,**
daß über mindestens 30 % der Fermentationsdauer die Konzentration an verwertbaren Zucker im Fermentationsmedium auf max. 0,3 Gew.-% gehalten wird.

14. Tierfuttermittel oder Prämix,
**gekennzeichnet durch**
einen Gehalt an Aminosäuretierfuttermittelsupplement gemäß einem der vorhergehenden Ansprüche.

15. Verwendung des Aminosäuretierfuttersupplements gemäß einem der Ansprüche 1 bis 6 oder hergestellt gemäß einem der Ansprüche 7 bis 13 zur Supplementierung bzw. Herstellung eines Tierfuttermittels oder Prämixes.

**Claims**

1. Amino acid animal feed supplement based on fermentation broth,
characterised in that
the amino acid supplement still contains the predominant part of the constituents of the fermentation broth and the dry mass has the following composition:

EP 0 533 039 B1

| Amino acid(s) | 40 to 90% by weight |
|---|---|
| Proteins max. | 10% by weight |
| Carboxylic acids with fewer than 8 C atoms max. | 8% by weight |
| Total sugar max. | 10% by weight |
| Fats and oils max. | 5% by weight |
| Mineral matter | 3 to 30% by weight, |

and that the amino acid supplement can be isolated directly from the fermentation broth by spray drying.

2. Supplement according to claim 1,
characterised in that
a part of the biomass is removed.

3. Supplement according to one of the preceding claims,
characterised in that
the water content is from 0.5 to 5% by weight.

4. Supplement according to one of the preceding claims,
characterised in that
up to 10% by weight of fermentation biomass is contained in the dry mass.

5. Supplement according to one of the preceding claims,
characterised in that
the content of fermentatively produced amino acid(s) is at least 49.5% by weight.

6. Supplement according to one of the preceding claims,
characterised in that
the content of fermentatively produced amino acid is at most 60% by weight in the case of basic amino acid or 85% by weight in the case of neutral amino acids.

7. Process for the production of an amino acid animal feed supplement based on fermentation broth, comprising the following steps:
   - cultivation of a microorganism producing at least one α-amino acid in a fermentation medium which contains at least one source of carbon, at least one source of nitrogen, mineral salts and trace elements,
   - conducting the fermentation so that at the end thereof a crude fermentation broth having a content of utilisable sugar of at most 4 g/l is obtained,
   - at least partial reduction of the microorganisms forming the biomass, in particular to a maximum of 10% by weight in the dry mass,
   so that the amino acid animal feed supplement can be isolated directly from the fermentation broth by spray drying.

8. Process for the production of an amino acid animal feed supplement based on fermentation broth, comprising the following steps:
   - cultivation of a microorganism producing at least one α-amino acid in a fermentation medium which contains at least one source of carbon, at least one source of nitrogen, mineral salts and trace elements,
   - conducting the fermentation so that at the end thereof a crude fermentation broth having a content of utilisable sugar of at most 4 g/l is obtained,
   - at least partially decreasing the microorganisms forming the biomass, in particular to a maximum of 10% by weight in the dry mass, and optionally other substances by means of mechanical separation techniques while leaving the predominant proportions of the remaining components of the fermentation broth in the supplement,
   so that the amino acid animal feed supplement can be isolated directly from the fermentation broth by spray drying.

13

9. Process for the production of an amino acid animal feed supplement based on fermentation broth according to one of claims 7 or 8, whereby the fermentation is conducted in such a way that at the end thereof a crude fermentation broth is obtained having a content of utilisable sugar of at maximum 4 g/l and a content of biomass which is limited so that the dry mass of the fermentation broth contains a maximum of 10% by weight of protein and in particular from 40 to 90% by weight of amino acid(s), a maximum of 8% by weight of carboxylic acids having fewer than 8 C atoms, a maximum of 10% by weight of total sugar, a maximum of 5% by weight of fats and oils and from 3 to 30% by weight of mineral substances.

10. Process according to one of claims 7 to 9,
characterised in that
the crude fermentation broth finally has a solids content of from 7.5 to 26% by weight.

11. Process according to one of claims 7 to 10,
characterised in that
the fermentation broth is subsequently dried to a water content of less than or equal to 5% by weight.

12. Process according to one of claims 7 or 11,
characterised in that
the fermentation is conducted so that at the end thereof the crude fermentation broth contains from 1 to 20% by weight of amino acid(s).

13. Process according to one of claims 7 to 12,
characterised in that
for at least 30% of the fermentation time the concentration of utilisable sugar is maintained at a maximum of 0.3% by weight.

14. Animal feed or premix,
characterised by
having a content of amino acid animal feed supplement according to one of the preceding claims.

15. Use of the amino acid animal feed supplement according to one of claims 1 to 6 or produced according to one of claims 7 to 13 for supplementing or producing an animal feed or premix.

## Revendications

1. Additif pour l'alimentation des animaux à base de moût de fermentation, caractérisé en ce que le supplément d'aminoacide contient encore la partie principale des composants du moût de fermentation qui comporte la composition suivante de masse sèche :

| | |
|---|---|
| Aminoacide(s) | 40-90 % en poids |
| Protéines max. | 10 % en poids |
| Acides carboxyliques avec moins de 8 atomes de C max. | 8 % en poids |
| Sucre total max. | 10 % en poids |
| Graisses et huiles max. | 5 % en poids |
| Matières minérales | 3-30 % en poids |

et en ce que l'additif d'aminoacide peut être obtenu directement par séchage par pulvérisation à partir du moût de fermentation.

2. Additif selon la revendication 1, caractérisé en ce qu'une partie de la biomasse est enlevée.

3. Additif selon une des revendications précédentes, caractérisé en ce que la teneur en eau se situe de 0,5 à 5 % en poids.

4. Additif selon une des revendications précédentes, caractérisé en ce qu'il contient dans la masse sèche jusqu'à 10 % en poids de biomasse de fermentation.

EP 0 533 039 B1

**5.** Additif selon une des revendications précédentes, caractérisé en ce que la teneur en aminoacide(s) obtenu(s) par fermentation se monte au moins à 49,5 % en poids.

**6.** Additif selon une des revendications précédentes, caractérisé en ce que la teneur en aminoacide préparé par fermentation se monte au maximum à 60 % en poids dans le cas d'un aminoacide basique ou à 85 % en poids dans le cas d'aminoacides neutres.

**7.** Procédé de préparation d'un additif d'aminoacide pour alimentation des animaux à base de moût de fermentation, avec les étapes :
- culture d'un micro-organisme produisant au moins un aminoactde α dans un milieu de fermentation, qui contient au moins une source de carbone, au moins une source d'azote, des sels minéraux et des substances en traces,
- conduite de la fermentation de manière qu'à sa fin on obtienne un moût de fermentation brut ayant une teneur en sucre utile d'au maximum 4 g/l,
- réduction au moins partielle des micro-organismes formant la biomasse en particulier au maximum à 10 % en poids dans la masse sèche,
de façon que l'additif aminoacide d'alimentation pour animaux puisse être obtenu directement par séchage par pulvérisation à partir du moût de fermentation.

**8.** Procédé de préparation d'un additif aminoacide d'alimentation pour animaux à base de moût de fermentation, avec les étapes :
- culture d'un micro-organisme produisant au moins un aminoacide α dans un milieu de fermentation qui contient au moins une source de carbone, au moins une source d'azote, des sels minéraux et des substances en traces,
- conduite de la fermentation de manière qu'à sa fin on obtienne un moût de fermentation brut ayant une teneur en sucre utile d'au minimum 4 g/l,
- réduction au moins partielle des micro-organismes formant la biomasse en particulier au maximum à 10 % en poids dans la masse sèche et éventuellement d'autres substances par des techniques de séparation mécaniques et en laissant les proportions principales des composants restant dans l'additif,
de sorte que l'additif aminoacide d'alimentation pour animaux peut être obtenu directement par séchage par pulvérisation à partir du moût de fermentation.

**9.** Procédé de préparation d'un additif aminoacide d'alimentation pour animaux, à base de moût de fermentation selon une des revendications 7 ou 8, dans lequel la fermentation est conduite de manière qu'à sa fin on obtienne un moût de fermentation brut ayant une teneur de sucre utile au maximum de 4 g/l et une teneur en biomasse qui est limitée pour que la masse sèche du moût de fermentation contienne au maximum 10 % en poids de protéine et en particulier de 40 à 90 % d'aminoacide (s), au maximum à 8 % en poids d'acides carboxyliques ayant moins de 8 atomes de C, au maximum à 10 % en poids de sucre total, au maximum à 5 % en poids de graisses et d'huiles et de 3 à 30 % en poids de matières minérales.

**10.** Procédé selon une des revendications 7 à 9, caractérisé en ce qu'à la fin dans le moût de fermentation brut, existe une teneur en matière solide de 7,5 à 26 % en poids.

**11.** Procédé selon une des revendications 7 à 10, caractérisé en ce que le moût de fermentation est ensuite amené par séchage à une teneur en eau ≤ 5 % en poids.

**12.** Procédé selon une des revendications 7 ou 11, caractérisé en ce que la fermentation est conduite de manière qu'à sa fin dans le moût brut de fermentation l' (les) aminoacide(s) soient présents pour 1 à 20 % en poids.

**13.** Procédé selon une des revendications 7 à 12, caractérisé en ce que sur au moins 30 % de la durée de la fermentation la concentration en sucre utile est maintenue dans le milieu de fermentation au maximum à 0,3 % en poids.

**14.** Agent d'alimentation pour animaux ou prémélange caractérisé par une teneur en additif aminoacide d'alimentation pour animaux selon une des revendications précédentes.

15

15. Utilisation de l'additif aminoacide d'alimentation pour animaux selon une des revendications 1 à 6 ou préparé selon une des revendications 7 à 13 pour l'addition ou la préparation d'un agent d'alimentation pour animaux ou pré-mélange.